# EUROPÄISCHE PATENTSCHRIFT

(11) **EP 4 146 288 B1**
(45) Veröffentlichungstag und Bekanntmachung des Hinweises auf die Patenterteilung: **25.06.2025**
(21) Anmeldenummer: 21726058.7
(22) Anmeldetag: 05.05.2021
(51) Int. Cl.: A61L 2/10, A61L 2/18, A61L 2/22, A61L 2/24, B65G 47/52

(54) **DESINFEKTIONSEINRICHTUNG FÜR TRANSPORTBEHÄLTNISSE EINER INSPEKTIONSANORDNUNG**
DISINFECTION DEVICE FOR TRANSPORT CONTAINERS OF AN INSPECTION ARRANGEMENT
DISPOSITIF DE DÉSINFECTION DES CONTENEURS DE TRANSPORT D'UN DISPOSITIF D'INSPECTION

(30) Priorität: 07.05.2020 DE 102020112465
(43) Veröffentlichungstag der Anmeldung: 15.03.2023
(73) Patentinhaber: Smiths Detection Germany GmbH, 65205 Wiesbaden (DE)
(72) Erfinder: HESS, Gregor, 65205 Wiesbaden (DE)
(74) Vertreter: Klunker IP Patentanwälte PartG mbB
(86) Internationale Anmeldenummer: PCT/EP2021/061870
(87) Internationale Veröffentlichungsnummer: WO 2021/224330

(56) Entgegenhaltungen:
- EP-A1- 1 151 919
- EP-A1- 1 588 720
- EP-A1- 1 588 720
- CN-U- 207 046 320
- KR-A- 20200 017 996
- KR-A- 20200 017 996
- US-A1- 2008 289 649
- US-A1- 2008 289 649
- US-A1- 2015 115 172
- US-A1- 2015 190 538
- US-A1- 2015 190 538
- SANYCAR SRL, 25 March 2020 (2020-03-25), Retrieved from the Internet <URL:https://italiavola.com/2020/03/25/laeroporto-di-cagliari-e-il-primo-scalo-in-europa-ad-aver-adottato-un-nuovo-sistema-di-disinfezione/> [retrieved on 20240221]
- SANYCAR SRL, 3 May 2020 (2020-05-03), Retrieved from the Internet <URL:https://italiavola.com/2020/05/03/sanycar-annuncia-un-accordo-di-partnership-strategica-con-la-britannica-uv-technology-global/> [retrieved on 20240221]

## Beschreibung

### Technisches Gebiet

Die vorliegende Offenbarung betrifft eine Inspektionsanordnung zur Überprüfung von Inspektionsobjekten, wie beispielsweise Handgepäck und anderen von Personen mitgeführten Gegenständen, mit einer Anordnung zur automatischen Desinfektion von Transportbehältnissen, die an der Inspektionsanordnung zur Aufnahme von Inspektionsobjekten eingesetzt werden.

### Hintergrund der vorliegenden Offenbarung

Zur Sicherheitskontrolle von Handgepäck und anderen von Personen mitgeführten Gegenständen, beispielsweise bei der Sicherheitskontrolle an Checkpoints auf Flughäfen, werden bekannterweise Inspektionsanlagen zur zerstörungsfreien Inspektion, beispielsweise Röntgeninspektionsanlagen verwendet, in denen die mitgeführten Gegenstände als Inspektionsobjekte mit Röntgenstahlen bildgebend durchstrahlt werden. Ziel der Inspektion ist primär das Auffinden von Gefahrengegenständen, wie Waffen oder Sprengmittel, aber auch illegalen Gegenständen, wie Drogen oder Schmuggelwaren.

Beispielsweise zeigt die DE 10 2006 006208 A1, dass die Inspektionsobjekte auf einer Fördereinrichtung, üblicherweise einem Transportband, durch die Inspektionsanlage gefördert werden, die von einer Bedienperson bedient wird. Üblicherweise ist an einer Eingangsseite vor dem Förderer eine Auflagestelle für die Inspektionsobjekte angeordnet, die üblicherweise als Bandförderer ausgestaltet ist. Entsprechend befindet sich an der Ausgangsseite hinter der Inspektionsanlage eine Entnahmestelle, an der die geprüften Inspektionsobjekte entnommen werden können. Beispielsweise kann eine leicht abschüssige Rollenbahn von der Transporteinrichtung zu der Entnahmestelle führen, auf der die geprüften Inspektionsobjekte sich selbsttätig zu der Entnahmestelle bewegen oder einfach manuell bewegt werden können.

Für den Transport kleiner Inspektionsobjekte (wie beispielsweise Geldbörsen, Mobiltelefone, Laptops, kleine Rucksäcke etc. und Kleidungstücke werden bekannterweise Transportbehältnisse, beispielsweise in Form von Transportwannen, verwendet, in die Inspektionsobjekte gelegt werden. Wie größere Inspektionsobjekte (beispielsweise Taschen, Trolleys, Handkoffer etc.) werden die Transportbehältnisse mit den darin befindlichen Inspektionsobjekten an einer Auflagestelle positioniert, der Transporteinrichtung übergeben und von dieser durch die Inspektionsanlage gefördert. Nach der zerstörungsfreien Inspektion werden die geprüften und unverdächtigen Inspektionsobjekte an der Entnahmestelle von deren Eigentümer, beispielsweise einem Passagier, wieder aus dem Transportbehältnis entnommen. Nach dem Entleeren werden die Transportbehältnisse wieder zum Eingang der Inspektionsanordnung zurückgebracht. Dazu werden sie entweder durch das Bedienungspersonal zurückgetragen oder an die Auflagestelle durch ein motorisiertes Rückführsystem zurückgefördert.

Im Laufe des Betriebs werden an einem Checkpoint eines Flughafens die Transportbehältnisse mit unterschiedlichen Gegenständen von verschiedenen Personen befüllt und entleert. Neben elektronischen Geräten und kleinen Taschen, sind die Passagiere angehalten, auch Flüssigkeiten, chemische Substanzen wie Kosmetikprodukte oder Medikamente, und sogar ihre eigenen getragenen und damit schmutzigen Schuhe (z.B. Stiefel) in die Transportbehältnisse für deren Inspektion zu legen.

Durch die hohe Anzahl von Personen, die die Transportbehältnisse verwenden bzw. berühren, sind die Oberflächen der Transportbehältnisse einer hohen Kontaminierung mit Verunreinigungen, aber auch Mikroorganismen ausgesetzt. Eine Fachstudie hat gezeigt, dass die Oberfläche von Transportbehältnissen an Flughäfen beispielsweise Grippeviren A und B, Coronaviren sowie Adeno- und Rhinoviren aufweisen und damit ein Vektor für die Übertragung der zugehörigen Krankheiten sein kann. Damit wären Checkpoints an Flughäfen nicht nur Verkehrsknotenpunkt, sondern möglicherweise auch ein Hotspot für die Ausbreitung von Krankheitserregen.

Die derzeit übliche Maßnahme, um diese Gefahr zu mindern, besteht darin, die Transportbehältnisse regelmäßig aus dem Einsatz am Checkpoint herauszunehmen und diese manuell anderorts zu reinigen und zu desinfizieren. Dies ist keine perfekte Lösung. Da jeder Checkpoint eine begrenzte Anzahl von Transportbehältnissen hat, wäre es nicht praxistauglich, jede einzelne Transportwanne nach jeder Verwendung zu desinfizieren, ohne den reibungslosen Ablauf und den Durchsatz an einem Checkpoint zu stark zu beeinträchtigen.

DE 10 2006 006 208 B4 und DE 10 2010 026 940 A1 derselben Anmelderin zeigen Beispiele für an sich bekannte Inspektionsanordnungen mit wenigstens einer Inspektionsanlage sowie Transporteinrichtungen für die oben genannten Transportbehältnisse zur Aufnahme von Inspektionsobjekten. KR 2020 0017996 A zeigt eine Desinfektionsvorrichtung zur automatischen Desinfektion von Transportbehältern. US 2008/0289649 A1 betrifft eine tragbare Desinfektionsvorrichtung für tragbare Geräte wie einen Einkaufswagen. EP 1 588 720 A1 bezieht sich auf die Desinfektion von Menschen oder Teilen davon oder von Gegenständen wie Dokumenten, Geld, Gläsern usw. US 2015/0190538 A1 zeigt, ähnlich wie EP 1 588 720 A1, eine Vielzahl von Ausführungsformen von tragbaren oder installierten Desinfektionsvorrichtungen.

EP 1 151 919 A1 zeigt Vorrichtung zur Rückführung von Behältern an einer Inspektionsanlage. Die Vorrichtung besteht aus einem Stützgestell und einem an dem Stützgestell vorgesehenen Führungsrahmen für die Rückführung der Behälter, wobei der Führungsrahmen oberhalb der Inspektionsanlage zur Kontrolle von Gepäckstücken angeordnet ist. Ähnliches zeigt die CN 207 046 320 U zeigt ein Gepäckinspektionssystem mit einer Behälter-Rückholvorrichtung mit einem Förderband, das Behälter unter einer Inspektionsanlage vom Ende des Gepäckinspektionssystems zu einer Ausgabestelle am Zugang des Gepäckinspektionssystems transportiert.

US 2015/115172 A1 zeigt eine Vorrichtung zum Desinfizieren von in der Hand gehaltenen Geräten, die jeweils eine Oberfläche aufweisen, die händisch berührt wird, wenn das Gerät in Gebrauch ist. Die Desinfektionsvorrichtung besteht aus: einer Quelle für desinfizierende Strahlung; und einem Fördersystem, das die Geräte an der Quelle vorbeiführt, wobei die Ober- und/oder Unterseiten jedes Geräts der Quelle zugewandt sind, wobei das Fördersystem aus einer Vielzahl von hohlen, transparenten Rollen besteht; und die Quelle für desinfizierende Strahlung eine Vielzahl von Lampen aufweist, die jeweils in einer der trans parentalen Rollen untergebracht sind.

Inspektionsanordnungen mit einem Desinfektionssystem für die Desinfektion von Handgepäckbehältern sind auch auf den folgenden Webseiten zu finden: https:// italiavola.com/2020/03/25/laeroporto-di-cagliari-e-il-primo-scalo-in-europa-ad-aver-adottato-un-nuovo-sistema-di-disinfezione/ und https://italiavola.com/2020/05/03/sanycar-annuncia-un-accordo-dipartnership-strategica-con-la-britannica-uv-technology-global/

### Allgemeine Beschreibung der vorliegenden Offenbarung

Es ist somit eine Aufgabe, eine Lösung vorzuschlagen, vermittels der die oberflächliche Belastung von Transportbehältnisse, die bei einer Inspektionsanordnung zur Überprüfung von Inspektionsobjekten, wie beispielsweise Handgepäck und anderen von Personen mitgeführten Gegenständen, eingesetzt werden, ohne Beeinträchtigung des Betriebs an der Inspektionsanordnung (Checkpoint) möglichst über dem Zeitverlauf auf einem gleichen niedrigen Niveau zu halten und somit die Gefahr der Ausbreitung von Krankheitserregern und Keimen zu verhindern oder zumindest zu mindern.

Die Aufgabe wird mit den Merkmalen einer Inspektionsanordnung gemäß dem unabhängigen Anspruch 1 gelöst. Weitere Vorteile und Ausführungen ergeben sich aus den Unteransprüchen, der Beschreibung und den Zeichnungen.

Ein Kerngedanke der hier vorgeschlagenen Lösung liegt darin, die oberflächlichen Mikroben und Keime jeglicher Art an oder auf den Transportbehältnissen der Inspektionsanordnung im laufenden Betrieb zu verringern, indem die Transportbehältnisse regelmäßig, wahlweise oder bedarfsgesteuert parallel zu den laufenden Sicherheitskontrollen automatisch desinfiziert werden.

Die Erfindung stellt eine Inspektionsanordnung zur Überprüfung von Inspektionsobjekten, wie beispielsweise Handgepäck und anderen von Personen mitgeführten Gegenständen, bereit, mit: einer Auflagestelle, auf die ein Transportbehältnis gelegt und mit einem oder mehreren Inspektionsobjekten befüllt werden kann; einer Entnahmestelle, an der das eine oder die mehreren Inspektionsobjekte aus dem Transportbehältnis entnommen werden kann oder können; einer Inspektionsanlage, beispielsweise einer Röntgeninspektionsanlage, zur zerstörungsfreien Inspektion des einen oder der mehreren Inspektionsobjekte, wobei die Inspektionsanlage zwischen der Auflagestelle und der Entnahmestelle angeordnet ist; einer sich durch die Inspektionsanlage erstreckenden Fördereinrichtung zum Fördern der Transportbehältnisse durch die Inspektionsanlage entlang einer Hinstrecke, die sich von der Auflagestelle bis zur Entnahmestelle erstreckt; und eine Desinfektionseinrichtung zur automatischen Desinfektion der Transportbehältnisse.

Die Desinfektionseinrichtung dient zur automatischen Desinfektion der Transportbehältnisse für den Transport durch die Inspektionsanlage und zur zerstörungsfreien Inspektion in der Inspektionsanlage von einem oder mehreren Inspektionsobjekten. Mit anderen Worten dient das Transportbehältnis zur Aufnahme der einen oder mehreren Inspektionsobjekte und anschließend als Behältnis für den Transport durch die Inspektionsanlage. Die Desinfektionseinrichtung weist auf: eine Eingangsstelle, an der geleerte Transportbehältnisse ankommen und die mit der Entnahmestelle der Inspektionsanordnung, an der inspizierte Inspektionsobjekte aus den Transportbehältnis entnommen werden können, verbunden ist; eine Ausgangsstelle, die mit der Auflagestelle der Inspektionsanordnung, auf der die Transportbehältnisse abgestellt und mit einem oder mehreren Inspektionsobjekten befüllt werden können, verbunden ist; und mindestens eine Rückfördereinrichtung zum Befördern der geleerten Transportbehältnisse über eine Rückstrecke, die sich von der Eingangsstelle bis zur Ausgangsstelle erstreckt. Die hier vorgeschlagene Lösung besteht in wenigstens einer an der Rückstrecke angeordneten automatischen Desinfektionsvorrichtung zur Desinfektion der geleerten Transportbehältnisse.

Erfindungsgemäß ist die entsprechend in eine vorhandene Inspektionsanordnung zur Gepäckprüfung - wie z.B. bei der Kontrolle von Handgepäck - integriert, um die erfindungsgemäße Inspektionsanordnung zu bilden. Der Vorteil besteht dabei darin, dass eine bereits vorhandene Inspektionsanordnung durch Hinzufügung der Desinfektionsvorrichtung verbessert werden kann, da dann die Transportbehältnisse im laufenden Einsatz desinfiziert werden können. Eine Herausnahme oder ein Austausch von Transportbehältnisses zwecks Reinigung ist dann nicht mehr erforderlich. Somit wird der Betriebsablauf an der Inspektionsanordnung durch eine Handhabung zur Reinigung der Transportbehältnisse nicht mehr beeinträchtigt. Auch müssen weniger Transportbehältnisse vorgehalten werden, da sich diese dann im Wesentlichen im Dauereinsatz befinden.

Erfindungsgemäß weist die Desinfektionsvorrichtung wenigstens eine UV-Strahlungseinheit zum Bestrahlen der geleerten Transportbehältnisse auf. Die wenigstens eine Strahlungseinheit kann eine oder mehrere UV-Lichteinheiten sowie optional eine oder mehrere Reflektoreinheiten aufweisen, die für eine Bestrahlung der Oberflächen der Transportbehältnisse, bevorzugt der gesamten Innen- und Außenoberfläche der Transportbehältnisse angeordnet sind. Eine solche Konfiguration ermöglicht eine Bestrahlung der Transportbehältnisse aus unterschiedlichen Richtungen und garantiert eine komplette Desinfektion der gesamten Oberfläche.

Beispielsweise kann eine UV-Strahlungseinheit verwendet werden, die UV-C Strahlen erzeugt. Vorzugsweise beruht der Wirkmechanismus der UV-C-Entkeimung auf rein physikalischer und nicht auf chemischer Basis, wodurch eine Benetzung der Oberfläche der Transportbehältnisse mit einer Desinfektionslösung vermieden werden kann, wenn das nicht gewünscht ist.

Alternativ oder zusätzlich kann die Desinfektionsvorrichtung wenigstens eine Sprüheinheit mit mindestens einer Sprühdüsen aufweisen, die für ein Besprühen der Oberflächen der Transportbehältnisse mit einer Desinfektionslösung, bevorzugt der gesamten Innen- und Außenoberfläche der Transportbehältnisse eingerichtet ist.

Die Desinfektionslösung kann beispielsweise eine Lösung sein, die mindestens eines von einem Bakterizid, einem Fungizid, einem Sporizid und einem Viruzid oder eine beliebige Kombination dieser Bestandteile beinhaltet. Alternativ kann als Desinfektionslösung auch Wasserstoffperoxid (H2O2) verwendet werden; beispielsweise eine Lösung aus Wasser (H2O) mit 5-10%, bevorzugt 7-8% H2O2. Die Desinfektionslösung kann in einem oder mehrere Behälter vorgehalten werden, die durch einen Anschlussschlauch mit der wenigstens einen Sprühdüse verbunden ist. Die Sprüheinheit kann ferner eine entsprechende Pumpe aufweisen, um die Desinfektionslösung mit dem notwendigen Druck durch die Sprühdüse zu fördern, um ein gleichmäßiges und vollständiges Besprühen der Transportbehältnisse zu ermöglichen.

Alternativ oder zusätzlich kann die Desinfektionsvorrichtung einen Erhitzer zum Erhitzen der Desinfektionslösung aufweisen. Somit wird ein entsprechender Hochtemperaturdampf bestehend aus der Desinfektionslösung erzeugt, um die Desinfektionswirkung zu verbessern. Der Erhitzer kann dabei mit dem Behälter der Desinfektionslösung oder direkt mit den Einspritzdüsen in Verbindung stehen. Beispielsweise kann H2O2-Heißdampf als Desinfektionsmittel eingesetzt werden.

Alternativ oder zusätzlich kann die Desinfektion auch mittels Ultraschall aus der Desinfektionslösung erzeugtem Kaltdampf erfolgen. Auch hier kann als Desinfektionslösung eine Lösung aus H2O mit H2O2 zur Anwendung kommen.

D.h., die Sprüheinheit kann zur Erzeugung eines kalten oder heißen Sprühnebels oder eines kalten oder heißen Dampfs eingerichtet sein. Natürlich können die genannten Sprühvarianten auch in der Desinfektionsvorrichtung kombiniert werden. Beispielsweise kann in einem ersten Bereich eine Heißdampfdesinfektion und in einem anderen Bereich eine Kaltdampfdesinfektion stattfinden.

Die eine oder mehreren Sprüheinheiten können als alleinige Desinfektionsmaßnahme dienen oder mit der UV-Strahlungseinheit kombiniert werden. Damit wird die Desinfektionswirkung verbessert.

Die Desinfektionsvorrichtung kann ferner eine mechanische Reinigungseinheit aufweisen, beispielsweise ein Bürstensystem mit Bürsten aufweisen, die angeordnet sind, um auf die mit Desinfektionslösung besprühte Oberfläche der Transportbehältnisse mechanisch einzuwirken. Das Bürstensystem kann hierbei derart gestaltet werden, dass ein oder mehrere bewegliche Bürstenelemente (z.B. rotierende Bürsten) mit der Oberfläche der Transportbehältnisse in Kontakt stehen. Vorzugsweise ist das Bürstensystem mit der Sprüheinheit kombinierbar, um zum Beispiel zusätzlich Schmutz aus den Transportbehältnissen und von deren Oberflächen zu entfernen und gleichzeitig die Desinfektionslösung auf den Oberflächen besser zu verteilen.

Zusätzlich zur wenigstens einen Sprüheinheit kann die Desinfektionsvorrichtung ferner wenigstens eine Wärmestrahlungseinheit zum Bestrahlen und Verdampfen der auf der besprühten Oberfläche der Transportbehältnisse befindlichen Desinfektionslösung aufweisen.

Alternativ oder zusätzlich zur Wärmestrahlungseinheit kann die Desinfektionsvorrichtung ferner wenigstens eine Trocknungseinheit zum Abtrocknen von auf den Oberflächen der Transportbehältnisse verbliebener Desinfektionslösung aufweisen. Die wenigstens eine Trocknungseinheit kann mindestens ein Wärmegerät und/oder ein Gebläse, bevorzugt ein Heißluftgebläse aufweisen. Die Trocknungseinheit kann hinter oder neben der Sprüheinheit angeordnet werden.

Bevorzugt kann sich die Rückfördereinrichtung durch die Desinfektionsvorrichtung erstrecken oder durch diese hindurchführen.

Die Rückfördereinrichtung kann aktiv oder passiv ausgestaltet sein, oder als eine Kombination aus aktiver und passiver Rückförderung ausgestaltet sein. "Aktive Förderung" bedeutet, dass die Rückfördereinrichtung die Transportbehältnisse aktiv transportiert, beispielsweise mittels eines dazu angetriebenen Transportbands oder angetriebener Transportsegmente. "Passive Förderung" bedeutet, dass die Rückfördereinrichtung so ausgestaltet ist, dass die Transportbehältnisse sich selbsttätig vermittels Schwerkraft zurückbewegen; dazu kann die Rückfördereinrichtung beispielsweise als schiefe Ebene mit integrierten stationären Rollen ausgestaltet sein, sodass sich die Transportbehältnisse angetrieben durch die Schwerkraft in Richtung des Gefälles der schiefen Ebene bewegen.

Zusätzlich zu allen oben genannten Desinfektionsmaßnahmen kann die Desinfektionsvorrichtung ferner eine Kippeinrichtung oder Rotationseinrichtung aufweisen, die eingerichtet ist, die Lagestellung oder Anordnung der Transportbehältnisse während oder im Laufe eines Desinfektionsvorganges zu ändern. Die Kippeinrichtung kann zum Beispiel als ein oder mehrere nebeneinander angeordnete Vorsprünge gestaltet werden, die als Kippelemente der Kippeinrichtung bezeichnet werden können. Während sich ein Transportbehältnis auf dem Rückweg befindet, stößt diese an einem Kippelement an. Hierdurch wird die anfängliche Lagestellung des Transportbehältnis durch die verursachte Kippfunktion geändert. Mehrere Kippelemente können derart nacheinander angeordnet sein, sodass das Transportbehältnis schließlich die anfängliche Lagestellung wieder einnimmt und in Richtung der Ausgangsstelle bzw. Auflagestelle gelangt. Somit erfolgt eine 360-Grad-Desinfektion des Transportbehältnisses. Um die Bewegung er Rückfördereinrichtung nicht zu beeinträchtigen, ist vorzugsweise die Kippeinrichtung nicht direkt auf dem Rückförderer angebracht. Die Kippelemente können hierfür beispielsweise durch einen seitlichen Halter an der Anordnung fixiert werden, ohne den Bewegungsfluss der Rückfördereinrichtung zu behindern.

Erfindungsgemäß weist die Desinfektionsvorrichtung ferner eine Steuerungseinheit auf, die eingerichtet ist, die Desinfektionsvorrichtung in Abhängigkeit eines Kontaminierungsgrades der Oberflächen einzelner Transportbehältnisse zu steuern; dabei bedeutet "einzelner Transportbehältnisse", dass die Desinfektionsvorrichtung gezielt und damit selektiv bestimmte Transportbehältnisse gemäß einem vorbestimmten Kriterium desinfiziert.

Um einzeln identifiziert und damit selektiv desinfiziert werden zu können, können die einzelnen Transportbehältnisse mit einem berührungslos und somit auf Abstand erfassbaren Identifikationsmerkmal, beispielsweise einer optisch erkennbaren Kodierung (einer Bar- oder QR-Kodemarkierung) oder einer per Funk auslesbaren Identifikationseinheit (z.B. einem RFID-Transponder) ausgestattet sein. Damit kann beispielsweise die Einsatzhäufigkeit jedes Transportbehältnis individuell erfasst und ebenso eine individuelle Desinfektion/Reinigung, beispielsweise nach eine bestimmten Anzahl von Verwendungen, ausgelöst werden. "Gemäß einem festgelegten Ablauf" bedeutet, dass die Transportbehältnisse beispielsweise nach einem zuvor festgelegten zeitlichen Zyklus desinfiziert werden; beispielsweise nach einem vorbestimmten Zeitintervall, z.B. stündlich.

"Wahlweise aktiviert" bedeutet, dass die Desinfektion der Transportbehältnisse optional oder ausschließlich manuell von den Bedienpersonen der Inspektionsanordnung ausgelöst werden kann.

"In Abhängigkeit eines Kontaminierungsgrades der Oberflächen einzelner Transportbehältnisse" bedeutet, dass die Desinfektion und damit auch Reinigung der Transportbehältnisse in Abhängigkeit der optisch erkennbaren Verschmutzung der Oberflächen der Transportbehältnisse erfolgen kann.

Es versteht sich, dass die vorstehend genannten Prinzipien einzeln oder in beliebiger Kombination und ggf. modifiziert und natürlich mit weiteren Strategien verwendet werden können.

In der hier vorgeschlagenen Inspektionsanordnung erstreckt sich die Rückfördereinrichtung durch die Desinfektionsvorrichtung. Somit können die Transportwannen desinfiziert werden, während sich diese in Richtung der Ausgangsstelle bzw. Auflagestelle entlang der Rückstrecke bewegen.

Bei der Inspektionsanordnung kann sich die Rückstrecke wenigstens teilweise unterhalb der durch die Inspektionsanlage führenden Fördereinrichtung erstrecken. Ferner kann die Inspektionsanordnung eine Liftvorrichtung aufweisen, welche eingerichtet ist, die Transportbehältnisse von der Rückstrecke zu einer parallel zu der Auflagestelle verlaufenden Übergabestrecke für die Transportbehältnisse zu heben.

Die Desinfektionsvorrichtung der Inspektionsanordnung kann an der Rückstrecke unterhalb der Fördereinrichtung und/oder in der Liftvorrichtung angeordnet sein. Beispielsweise kann die Liftvorrichtung einen vertikal beweglichen Hebeboden nach Art eines Paternosters zum Heben der Behältnisse auf die Höhe der Übergabestrecke zurück aufweisen.

In einer alternativen Ausführung kann die Rückstrecke neben der und auf Höhe der durch die Inspektionsanlage führenden Fördereinrichtung verlaufen. Wie oben ausgeführt, kann ein Teil der Rückförderstrecke auch so angelegt sein, dass die Transportbehältnisse per Schwerkraft selbständig zurücklaufen. Die Rückstrecke kann auch seitlich an der Inspektionsanlage vorbei zurück zur Auflagestelle der Inspektionsanordnung verlaufen.

Die Transportbehältnisse sind bevorzugt Transportwannen. Des Weiteren sind die Transportwannen bevorzugt aus einem Kunststoff hergestellt. Wie oben angemerkt, können Transportbehältnisse jeweils ein kontaktlos erfassbares Identifikationselement aufweisen, wie beispielsweise eine optisch erfassbare Kodierung und/oder einen mittels Funk auslesbaren RFID-Transponder.

Abschließend sei angemerkt, dass die vorstehend beschriebenen Desinfektionsmaßnahmen allesamt geeignet kombiniert werden können.

Zunächst bietet sich an, die Desinfektionsvorrichtung unterhalb der Fördereinrichtung der Hinstrecke anzuordnen. Alternativ oder zusätzlich dazu, kann die Desinfektionsvorrichtung in der Lifteinrichtung angeordnet sein. Dabei können Teile der Desinfektionsvorrichtung unterhalb der Fördereinrichtung der Hinstrecke und andere Teile in der Lifteinrichtung angeordnet werden. Die UV-Strahlungseinheit und/ oder die Sprüheinheit können beispielsweise auf der Rückstrecke unterhalb der Inspektionsanlage und die Trockeneinheit direkt in der Lifteinrichtung angeordnet werden. Wird die komplette Desinfektionsvorrichtung in der Lifteinrichtung positioniert, führt dies zu einer besonders kompakten modularen Anordnung, die besonders gut in bereits vorhandene Inspektionsanordnungen (Checkpoints) integrierbar ist.

Eine vorhandene Inspektionsanordnung ohne Desinfektionsvorrichtung für die Transportbehältnisse kann mit einem Verfahren verbesserte werden, mit dem Schritt Integrieren einer Desinfektionsvorrichtung in eine Rückstecke zur Rückführung von Transportbehältnisses vom Bereich hinter der Inspektionsanlage zum Bereich vor der Inspektionsanlage.

### Kurzbeschreibung der Zeichnungsfiguren

Weitere Vorteile, Merkmale und Einzelheiten der vorliegenden Offenbarung ergeben sich aus der nachfolgenden Beschreibung, in der unter Bezugnahme auf die Zeichnungen bestimmte Ausführungsbeispiele im Einzelnen beschrieben sind. Dabei können die in den Ansprüchen und in der Beschreibung erwähnten Merkmale jeweils einzeln für sich oder in beliebiger Kombination wesentlich sein. Funktionsähnliche oder identische Bauteile oder Komponenten sind teilweise mit gleichen Bezugszeichen versehen. Die in der Beschreibung der Ausführungsbeispiele verwendeten Begriffe "links", "rechts", "oben", "unten" beziehen sich auf die Zeichnungen in einer Ausrichtung mit normal lesbaren Figurenbezeichnungen bzw. Bezugszeichen. Hierbei zeigen schematisch:
- Figur 1a: eine im Blockdiagram dargestellte Desinfektionseinrichtung;
- Figur 1b: eine im Blockdiagram dargestellte Inspektionsanordnung;
- Figur 2a: eine schematische Draufsicht auf eine mögliche Ausführung einer Inspektionsanordnung der Figur 1b;
- Figur 2b: eine schematische seitliche Ansicht der Inspektionsanordnung der Figur 2a;
- Figuren 3a-f: eine sequenzielle Darstellung einer Kippeinrichtung für die Desinfektionseinrichtung;
- Figur 4a: eine schematische Darstellung einer in eine Lifteinrichtung integrierten Strahlungseinheit; und
- Figur 4b: eine schematische Darstellung einer in eine Liftweinrichtung integrierten Sprüheinheit.

### Detaillierte Beschreibung der Ausführungsbeispiele

In der nachfolgenden Beschreibung werden zahlreiche spezifische Einzelheiten der hier offenbarten Lösung dargelegt. Es versteht sich jedoch, dass weitere Ausführungsformen auch ohne diese spezifischen Einzelheiten möglich sind. Dem Fachmann bekannte Schaltungen, Strukturen und Verfahren werden hier nicht im Detail besprochen, um das Verständnis der vorliegenden Beschreibung nicht unnötig zu erschweren. Begriffe "gekoppelt" und "verbunden/angeschlossen" sowie davon abgeleitete Begriffe werden hier nicht synonym verwendet. Bei spezifischen Ausführungsformen kann vielmehr "verbunden/angeschlossen" andeuten, dass zwei oder mehr Elemente in direktem physischem oder elektrischem Kontakt miteinander stehen. "Gekoppelt" kann bedeuten, dass zwei oder mehr Elemente zusammenwirken oder sich gegenseitig beeinflussen, wobei sie in direktem, aber auch indirektem physischem oder elektrischem Kontakt miteinander stehen können. Falls nicht anderweitig angegeben, deutet der Gebrauch der Ordinaladjektive "erster", "zweiter", "dritter" usw. zur Bezeichnung eines gemeinsamen Objekts lediglich an, dass auf verschiedene Beispiele von gleichartigen Objekten Bezug genommen wird, und soll nicht implizieren, dass die so bezeichneten Objekte in einer gewissen zeitlichen, räumlichen, Rangordnungs- oder sonstigen Reihenfolge auftreten müssen.

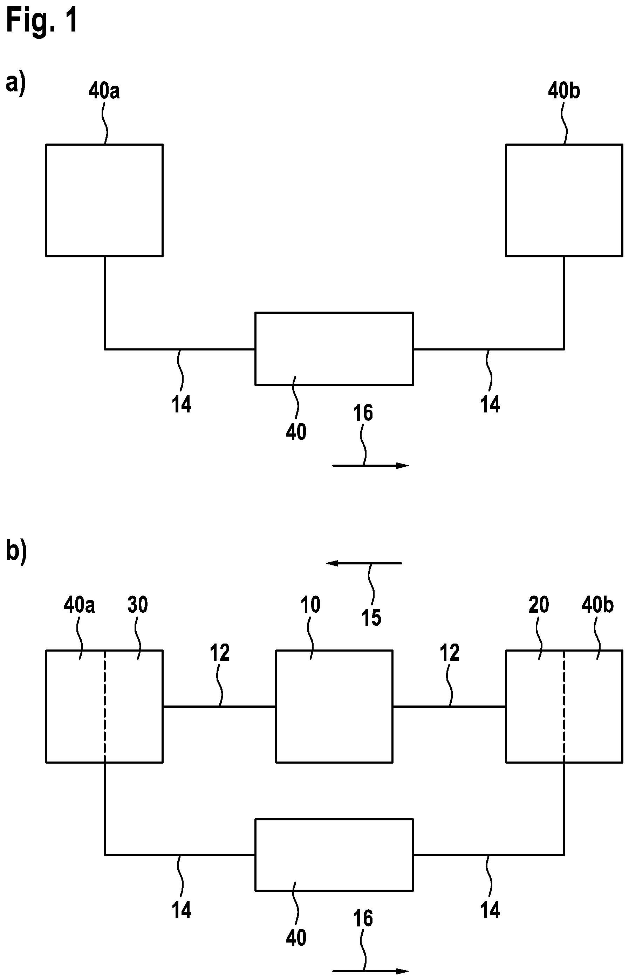

Figur 1a zeigt schematisch eine Blockdarstellung einer Desinfektionseinrichtung. Die Desinfektionseinrichtung zur automatischen Desinfektion von Transportbehältnissen 2 (in der Figur 1a nicht gezeigt und ab hier Transportwannen 2 genannt) weist eine Eingangsstelle 40a auf. An der Eingangsstelle 40a kommen die Transportwannen 2 geleert an, d.h. jegliche darin vorher abgelegte Inspektionsobjekte wurden zuvor entnommen (an der Entnahmestelle der Inspektionsanordnung, wie einem Checkpoint). Die geleerten Transportwannen 2 werden über eine Rückfördereinrichtung 14 entlang einer Rückstrecke 16 bis zu einer Ausgangsstelle 40b befördert. Entlang der Rückstrecke 16 ist eine Desinfektionsvorrichtung 40 vorgesehen, die für die Desinfektion der Transportwannen 2 eingerichtet ist.

Figur 1b zeigt schematisch eine Inspektionsanordnung zur Prüfung von Inspektionsobjekten, wie beispielsweise von einem Passagier an einem Checkpoint an einem Flughafen mitgeführtes Handgepäck und/oder andere kleine mitgeführte Gegenstände. In die Inspektionsanordnung kann die Desinfektionseinrichtung der Figur 1a so integriert werden, dass die Eingangsstelle 40a der Desinfektionseinrichtung mit der Entnahmestelle 30 der Inspektionsanordnung bzw. die Ausgangsstelle 40b der Desinfektionseinrichtung mit der Auflagestelle 20 der Inspektionsanordnung verbunden sind.

Bei der Figur 1b stehen die Eingangsstelle 40a und die Entnahmestelle 30 bzw. die Ausgangsstelle 40b und die Auflagestelle 20 direkt miteinander in Kontakt. Selbstverständlich könne diese auch durch einen oder mehrere Zwischenelemente (z.B. Transportband, Rollenbahn oder andere Geräte) miteinander verbunden werden. Hierbei wird eine in der Auflagestelle 20 vorhandene und mit Gegenständen befüllte Transportwanne 2 durch eine Inspektionsanlage 10 mittels einer Hinfördereinrichtung 12 hindurchgefördert. In der Inspektionsanlage 10 erfolgt eine Sicherheitskontrolle der Gegenstände als Inspektionsobjekte in einer an sich bekannten bildgebenden und zerstörungsfreien Weise beispielsweise vermittels Röntgeninspektion. Nachdem die in der Transportwanne 2 befindlichen Inspektionsobjekte geprüft wurden, erreichen diese über die Hinstrecke 15 die Entnahmestelle 30, an der die nun geprüften und für sicher befundenen Gegenstände vom Eigentümer wieder aus der Transportwanne 2 entnommen werden dürfen. Die geleerte Transportwanne 2 gelangt danach an die Eingangsstelle 40a der Desinfektionseinrichtung. Wie im Zusammenhang mit der Figur 1a bereits beschrieben wird die Transportwanne 2 auf ihren Rückweg zur Ausgangsstelle 40b mittels der Desinfektionsvorrichtung 40 desinfiziert. Die desinfizierte Transportwanne 2 gelangt danach an die Auflagestelle 20 zurück und steht desinfiziert für einen erneuten Arbeitszyklus zur Verfügung.

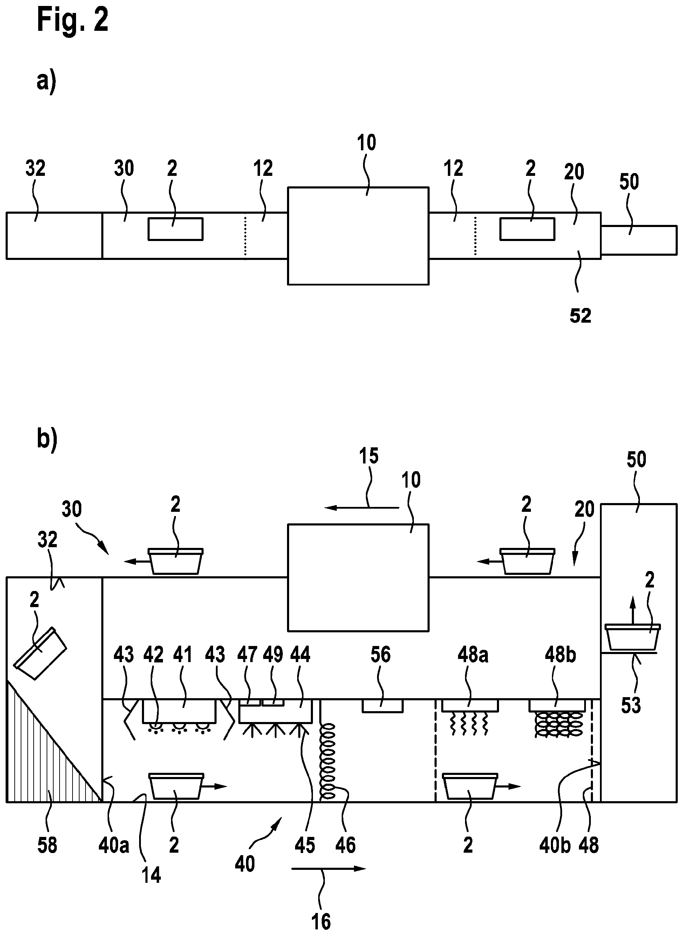

Figuren 2a und 2b zeigen eine Inspektionsanordnung (Checkpoint), wobei Figur 2a eine Draufsicht und die Figur 2b eine seitliche Ansicht der Inspektionsanordnung darstellen. Beispielsweise kann eine solche Inspektionsanordnung an einem Flughafen für die Sicherheitskontrolle von Handgepäck und anderen von Passagieren mitgeführten Gegenständen ihren Einsatz finden.

In der Figur 2a gelangen die Transportwannen 2 über einen vorgesehen Wannenlift 50 als Lifteinrichtung mittels einer Wannenübergabestrecke 52 zur Auflagestelle 20. Ab hier können Fluggäste ihre Gegenstände (z.B. Laptop, Schuhe, kleine Taschen, Geldbörse usw.) in den Transportwannen 2 deponieren und mittels der Hinfördereinrichtung 12 durch eine Inspektionsanlage 10 hindurchgefördert werden. Nach durchlaufener Sicherheitskontrolle mittels zerstörungsfreier und bildgebender Inspektion der Gegenstände als Inspektionsobjekte gelangt die Transportwannen 2 zur Entnahmestelle 30, an der die Fluggäste ihre geprüften und für sicher befundenen Gegenstände wieder aus der Transportwanne 2 entnehmen dürfen. Die zuvor benutzten und dann wieder geleerten Transportwannen 2 werden durch eine Rückstrecke 16 zur Auflagestelle 20 zurückgefördert.

Wie in der Figur 2b dargestellt, ist die Rückstrecke 16 unterhalb der Inspektionsanlage 10 angeordnet. Um von der Entnahmestelle 30 bis zur Eingangsstelle 40a zu gelangen, durchlaufen die Transportwannen 2 ein Zwischenelement hier als Rutschelement 58, welches mit einer Rutschstelle 32 versehen ist. Nun befinden sich die Transportwannen 2 auf den Rückfördereinrichtung 14 und werden durch eine Desinfektionsvorrichtung 40 geschleust.

Im dargestellten Beispiel sind in der Desinfektionsvorrichtung 40 verschiedene hintereinander angeordnete Desinfektionsabläufe vorgesehen, die innerhalb einer geschlossenen Struktur, beispielsweise einem Gehäuse, untergebracht sind. Die Struktur kann beispielsweise tunnelförmig sein und einen Eingang und einen Ausgang aufweisen.

Jeder einzelne Desinfektionsablauf ist einer bestimmten Einheit innerhalb der Desinfektionsvorrichtung 40 zugeordnet. Gemäß einer bestimmten Ausführung können diese Einheiten bildende Elemente oberhalb der Rückfördereinrichtung 14 oder direkt an der Decke der Struktur, beispielsweise dem Gehäuse, welche die Desinfektionsvorrichtung 40 bildet, angeordnet oder befestigt sein.

Im dargestellten Beispiel werden die Transportwannen 2 zuerst mittels UV-Lampen 42 einer Strahlungseinheit 41 bestrahlt. In der Strahlungseinheit 41 sind auch Reflektoren 43 vorgesehen, die eine Reflektion der UV-Strahlen rund um die Transportwannen 2 ermöglichen, sodass möglichst die gesamte Oberfläche der Transportwannen 2 erreicht wird.

Als nächstes passieren die Transportwannen 2 eine Sprüheinheit 44, in der die Transportwannen 2 mit einer Desinfektionslösung mit Hilfe von Sprühdüsen 45 besprüht werden. Die Desinfektionslösung befindet sich in einem Behälter 49, welcher mittels eines dazu gehörigen Erhitzers 47, der beispielsweise mit der Sprühdüse 45 zusammenwirkt, erhitzt werden kann. Somit kann die Desinfektionslösung dampfförmig (als Heißdampf) oder zumindest heiß zum Einsatz kommen, wodurch sich zum einen die Desinfektionswirkung erhöht, aber auch das Abtrocknen der Desinfektionslösung schneller von statten geht. Alternativ oder ergänzend kann die Sprüheinheit 44 auch zur Erzeugung von Kaltdampf, beispielsweise mittels Ultraschallzerstäubung eingerichtet sein.

Im gezeigten Beispiel ist zusätzlich ein optionales Bürstensystem 46 in der Sprüheinheit 44 angeordnet, um zum einen etwaige oberflächliche Verunreinigungen, wie Schmutzreste und/oder -partikel, von der Oberfläche der Transportwannen 2 zu entfernen, aber auch, um die Desinfektionslösung auf der Oberfläche der Transportwannen 2 besser zu verteilen.

Als nächstes durchlaufen die Transportwannen 2 eine Trocknungseinheit 48, die ein Wärmegerät 48a und nachfolgend ein Gebläse 48b vorsieht. Die Kombination aus Wärme und Luftstrom sorgen dafür, dass die Transportwannen 2 am Ausgang der Desinfektionsvorrichtung 40 möglichst trocken herauskommen.

Unmittelbar nach dem Ausgang der Desinfektionsvorrichtung 40, ist der Wannenlift 50 mit Hebeböden 53 angebracht, vermittels welcher die Transportwannen 2 wieder zur Auflagestelle 20 zurückgelangen.

Der Eingang bzw. Ausgang der Desinfektionsvorrichtung 40 entsprechen der Eingangsstelle 40a bzw. Ausgangsstelle 40b der Inspektionsanordnung. Die Desinfektionsvorrichtung 40 ist auch mit einer Steuerungseinheit 56 ausgestattet, die mit allen Einheiten (Strahlungseinheit 41, Sprüheinheit 44, Trocknungseinheit 48) verbunden ist und diese kontrolliert.

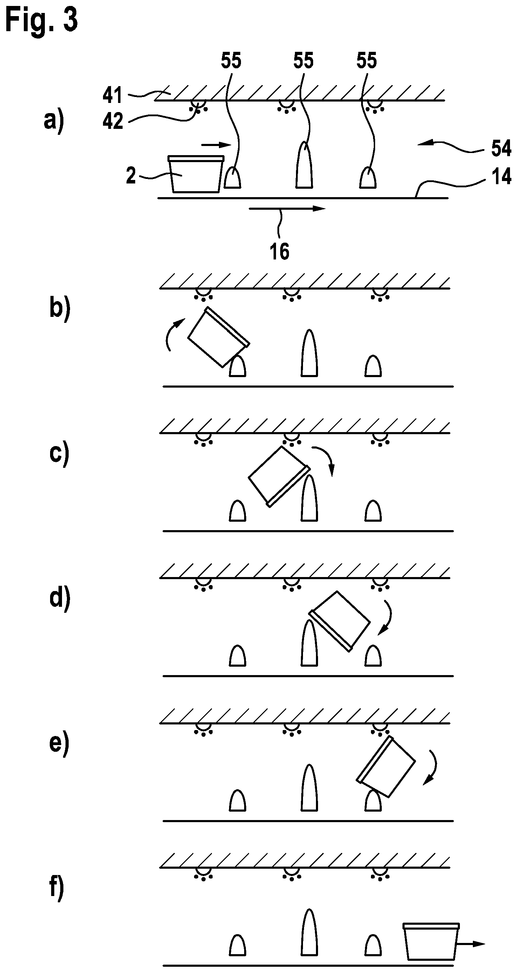

Figuren 3a bis 3f zeigen die Bewegung einer Transportwanne 2 innerhalb einer Strahlungseinheit 41, in die eine Kippeinrichtung 54 integriert ist. Die Kippeinrichtung 54 besteht hierbei aus mehreren nacheinander angeordneten Kippelementen 55, welche derart an der Struktur bzw. am Gehäuse der Desinfektionsvorrichtung 40 angebracht sind, dass diese Kippelemente 55 nicht die Laufrichtung der Rückfördereinrichtung 14 behindern. Die einzelnen Kippelemente 55 können sich voneinander durch verschiedene Formen und Größen unterscheiden, um das Umkippen bzw. die Änderung der Lagerung der Transportwanne 2 zu ermöglichen.

In den Figuren 3a bis 3f sind beispielsweise drei verschiedene Kippelemente 55 dargestellt, von denen das mittlere Element im Verhältnis zu den beiden äußeren Elementen höher ausgestaltet ist. Dies ermöglicht die komplette Rotation der Transportwanne 2, wie durch die Pfeile in den Figuren 3b bis 3e sichtbar gemacht wurde.

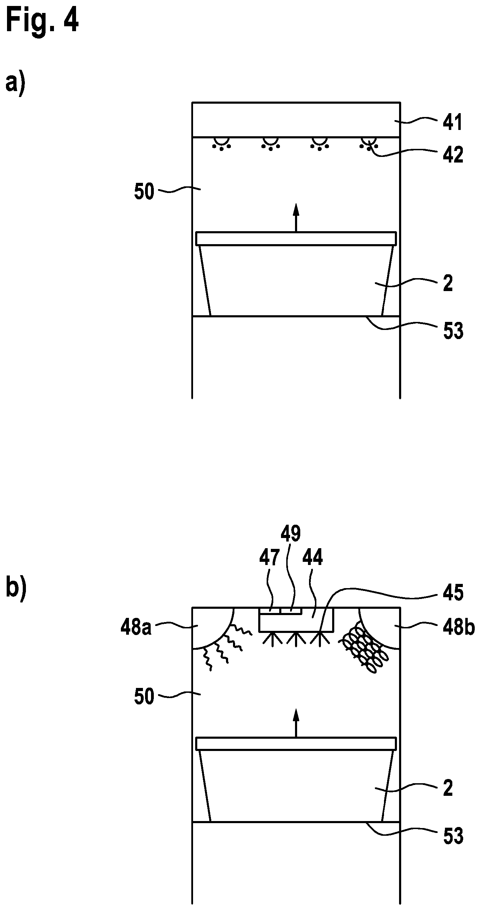

Figur 4a stellt ein Beispiel eines Wannenlifts 50 dar, in den eine Strahlungseinheit 41 integriert wurde. Die UV-Lampen 42 der Strahlungseinheit 41 sind hierbei an der Decke des Wannenlifts 50 angeordnet, um eine Bestrahlung der Transportwanne 2 während des Hochfahrens auf dem Hebeboden 53 zu ermöglichen.

Figur 4b stellt einen alternativen Wannenlift 50 dar, in den eine Sprüheinheit 44 integriert ist. Die Sprühdüsen 45 sind hierbei an der Decke des Wannenlifts 50 angeordnet, um eine Verteilung der Desinfektionslösung auf die Transportwanne 2 während des Hochfahrens auf dem Hebeboden 53 zu ermöglichen. Zusätzlich sind an der Decke des Wannenlifts 50 ein Wärmegerät 48a und ein Gebläse 48b zum Trocknen der Transportwannen 2 vorgesehen.

Über die Steuerungseinheit 56 werden die Sprüheinheit 44 und das Wärmegerät 48a sowie das Gebläse 48b zeitlich nacheinander gesteuert, so dass die Trocknung unmittelbar nach Beendigung der Besprühung der Transportwanne 2 stattfindet.

Falls die Desinfektionsvorrichtung 40 komplett im Wannenlift 50 integriert ist (vgl. Figuren 4a und 4b), entsprechen der Eingang und Ausgang des Wannenlifts 50 der Eingangsstelle 40a und der Ausgangsstelle 40b der Anordnung.

## Patentansprüche

1. Inspektionsanordnung zur Überprüfung von Inspektionsobjekten, wie beispielsweise Handgepäck und anderen von Personen mitgeführten Gegenständen, mit:
einer Auflagestelle (20), auf die ein Transportbehältnis (2) gelegt und mit einem oder mehreren Inspektionsobjekten befüllt werden kann;
einer Entnahmestelle (30), an der das eine oder die mehreren Inspektionsobjekte aus dem Transportbehältnis (2) entnommen werden kann,
einer Inspektionsanlage (10), beispielsweise eine Röntgeninspektionsanlage, zur zerstörungsfreien Inspektion des einen oder der mehreren Inspektionsobjekte, wobei die Inspektionsanlage (10) zwischen der Auflagestelle (20) und der Entnahmestelle (30) angeordnet ist,
einer sich durch die Inspektionsanlage (10) erstreckende Hinfördereinrichtung (12) zum Befördern der Transportbehältnisse (2) durch die Inspektionsanlage (10) entlang einer Hinstrecke (15), die sich von der Auflagestelle (20) bis zur Entnahmestelle (30) erstreckt, und
einer Desinfektionseinrichtung zur automatischen Desinfektion des Transportbehältnisses (2), welches für den Transport von dem einen oder den mehreren Inspektionsobjekten durch die Inspektionsanlage (10) und deren zerstörungsfreien Inspektion in der Inspektionsanlage (10) bestimmt ist,
wobei die Desinfektionseinrichtung aufweist:
eine Eingangsstelle (40a), an der geleerte Transportbehältnisse (2) ankommen und die mit der Entnahmestelle (30) der Inspektionsanordnung, an der inspizierte Inspektionsobjekte aus den Transportbehältnis (2) entnommen werden können, verbunden ist,
eine Ausgangsstelle (40b), die mit der Auflagestelle (20) der Inspektionsanordnung, auf der die Transportbehältnisse (2) abgestellt und mit dem einen oder mehreren Inspektionsobjekten befüllt werden können, verbunden ist, und
mindestens einer Rückfördereinrichtung (14) zum Befördern der geleerten Transportbehältnisse (2) über eine Rückstrecke (16), die sich von der Eingangsstelle (40a) bis zur Ausgangsstelle (40b) erstreckt, und
wenigstens eine an der Rückstrecke (16) angeordnete automatische Desinfektionsvorrichtung (40) zur Desinfektion der geleerten Transportbehältnisse (2),
wobei die Desinfektionsvorrichtung (40) wenigstens eine UV-Strahlungseinheit (42) zum Bestrahlen der geleerten Transportbehältnisse (2) aufweist, und **dadurch gekennzeichnet, dass**
die Desinfektionsvorrichtung (40) ferner eine Steuerungseinheit (56) aufweist, die eingerichtet ist, die Aktivierung der Desinfektionsvorrichtung (40) in Abhängigkeit eines Kontaminierungsgrades der Oberflächen einzelner Transportbehältnisse (2) zu steuern.

2. Inspektionsanordnung nach Anspruch 1, wobei die wenigstens eine Strahlungseinheit (41) eine oder mehrere UV-Lichteinheiten (42) sowie einen oder mehrere Reflektoreinheiten (43) aufweist, die für eine Bestrahlung der Oberflächen der Transportbehältnisse (2), bevorzugt der gesamten Innen- und Außenoberfläche der Transportbehältnisse (2) angeordnet sind.

3. Inspektionsanordnung nach Anspruch 1 oder 2, wobei die Desinfektionsvorrichtung (40) wenigstens eine Sprüheinheit (44) mit mindestens einer Sprühdüse (45) aufweist, die für ein Besprühen der Oberflächen der Transportbehältnisse (2) mit einer flüssigen oder dampfförmigen Desinfektionslösung, bevorzugt der gesamten Innen- und Außenoberfläche der Transportbehältnisse (2), eingerichtet sind.

4. Inspektionsanordnung nach Anspruch 3, wobei die Desinfektionsvorrichtung (40) ferner ein Bürstensystem (46) mit statischen und/oder rotierenden Bürsten aufweist, die angeordnet sind, um auf die mit Desinfektionslösung besprühte Oberfläche der Transportbehältnisse (2) mechanisch einzuwirken.

5. Inspektionsanordnung nach Anspruch 3 oder 4, wobei die Desinfektionsvorrichtung (40) ferner wenigstens eine Wärmestrahlungseinheit (47) zum Bestrahlen und Verdampfung der auf der besprühten oder bedampften Oberfläche der Transportbehältnisse (2) befindlichen Desinfektionslösung aufweist.

6. Inspektionsanordnung nach einem der Ansprüche 3 bis 5, wobei die Desinfektionsvorrichtung (40) ferner wenigstens eine Trocknungseinheit (48) zum Abtrocknen auf den Oberflächen der Transportbehältnisse (2) verbliebener Desinfektionslösung aufweist, wobei die wenigstens eine Trocknungseinheit (48) mindestens ein Wärmegerät (48a) und/oder ein Gebläse (48b), bevorzugt ein Heißluftgebläse aufweist.

7. Inspektionsanordnung nach einem der Ansprüche 1 bis 6, wobei sich die Rückfördereinrichtung (14) durch die Desinfektionsvorrichtung (40) erstreckt.

8. Inspektionsanordnung nach einem der Ansprüche 1 bis 7, wobei die Desinfektionsvorrichtung (40) ferner eine Kippeinrichtung (54) aufweist, um die Lagestellung der Transportbehältnisse (2) während eines Desinfektionsvorganges zu ändern.

9. Inspektionsanordnung nacheinem der Ansprüche 1 bis 8, wobei sich die Rückstrecke (16) wenigstens teilweise unterhalb oder neben der durch die Inspektionsanlage (10) führenden Hinfördereinrichtung (12) erstreckt und ferner eine Liftvorrichtung (50) aufweist, welche die Transportbehältnisse (2) von der Rückstrecke (16) zu einer parallel zu der Auflagestelle (20) verlaufenden Übergabestrecke (52) für die Transportbehältnisse (2) hebt.

10. Inspektionsanordnung nach Anspruch 9, wobei die Desinfektionsvorrichtung (40) an der Rückstrecke (16) unterhalb oder neben der Hinfördereinrichtung (12) und/oder in der Liftvorrichtung (50) angeordnet ist.

11. Inspektionsanordnung nach einem der Ansprüche 1 bis 8, wobei die Rückstrecke (16) ganz oder zum Teil als schiefe Ebene neben der durch das Prüfgerät (10) führenden Hinfördereinrichtung (12) verläuft und für eine Förderung der Transportbehältnisse (2) mittels Schwerkraft eingerichtet ist.

12. Inspektionsanordnung nach einem der Ansprüche 1 bis 11, wobei
die Transportbehältnisse (2) Transportwannen sind, und/oder
die Transportbehältnisse (2) jeweils ein kontaktlos erfassbares Identifikationselement aufweisen, wie beispielsweise eine optisch erfassbare Kodierung und/oder einen mittels Funk auslesbaren RFID-Transponder.

## Claims

1. Inspection arrangement for inspecting objects, such as hand luggage and other items carried by persons, comprising:
a lay-up point (20) on which a transport container (2) can be placed and filled with one or more objects to be inspected;
a pick-up point (30) at which the one or more inspection objects can be removed from the transport container (2),
an inspection apparatus (10), for example an X-ray inspection apparatus, for non-destructive inspection of the one or more inspection objects, the inspection apparatus (10) being arranged between the lay-up point (20) and the pick-up point (30),
a forwarding device (12) extending through the inspection apparatus (10) for conveying the transport containers (2) through the inspection apparatus (10) along an forward path (15) extending from the support point (20) to the pick-up point (30), and
a disinfection device for automatically disinfecting the transport container (2), which is intended for transporting the one or more inspection objects through the inspection apparatus (10) and for non-destructive inspection thereof in the inspection apparatus (10),
wherein the disinfection device comprises:
an input point (40a) at which emptied transport containers (2) arrive and which is connected to the pick-up point (30) of the inspection arrangement at which inspected inspection objects can be removed from the transport containers (2),
an output point (40b) which is connected to the lay-up point (20) of the inspection arrangement, at which the transport containers (2) can be placed and filled with the one or more inspection objects, and
at least one return device (14) for conveying the emptied transport containers (2) via a return path (16) extending from the input point (40a) to the output point (40b), and
at least one automatic disinfection device (40) arranged on the return path (16) for disinfecting the emptied transport containers (2),
wherein the disinfection device (40) has at least one UV radiation unit (42) for irradiating the emptied transport containers (2), and
**characterized in that** the disinfection device (40) further comprises a control unit (56) which is configured to control the activation of the disinfection device (40) as a function of a degree of contamination of the surfaces of individual transport containers (2).

2. Inspection arrangement according to claim 1, wherein the at least one radiation unit (41) comprises one or more UV light units (42) and one or more reflector units (43) which are arranged for irradiating the surfaces of the transport containers (2), preferably the entire inner and outer surfaces of the transport containers (2).

3. Inspection arrangement according to claim 1 or 2, wherein the disinfection device (40) has at least one spray unit (44) with at least one spray nozzle (45) which are arranged for spraying the surfaces of the transport containers (2) with a liquid or vaporous disinfectant solution, preferably the entire inner and outer surfaces of the transport containers (2).

4. Inspection arrangement according to claim 3, wherein the disinfection device (40) further comprises a brush system (46) with static and/or rotating brushes which are arranged to mechanically act on the surface of the transport containers (2) sprayed with disinfectant solution.

5. Inspection arrangement according to claim 3 or 4, wherein the disinfection device (40) further comprises at least one heat radiation unit (47) for irradiating and vaporizing the disinfectant solution located on the sprayed or vaporized surface of the transport containers (2).

6. Inspection arrangement according to one of claims 3 to 5, wherein the disinfection device (40) further comprises at least one drying unit (48) for drying disinfectant solution remaining on the surfaces of the transport containers (2), wherein the at least one drying unit (48) comprises at least one heating device (48a) and/or a fan (48b), preferably a hot-air fan.

7. Inspection arrangement according to one of claims 1 to 6, wherein the return device (14) extends through the disinfection device (40).

8. Inspection arrangement according to one of claims 1 to 7, wherein the disinfection device (40) further comprises a tilting device (54) for changing the position of the transport containers (2) during a disinfection process.

9. Inspection arrangement according to one of claims 1 to 8, wherein the return path (16) extends at least partially below or next to the forward conveyor device (12) leading through the inspection apparatus (10) and further comprises a lifting device (50) which lifts the transport containers (2) from the return section (16) to a transfer section (52) for the transport containers (2) extending parallel to the lay-up point (20).

10. Inspection arrangement according to claim 9, wherein the disinfection device (40) is arranged on the return path (16) below or next to the feed device (12) and/or in the lifting device (50).

11. Inspection arrangement according to one of claims 1 to 8, wherein the return section (16) extends wholly or partly as an inclined plane adjacent to the forward conveyor (12) leading through the testing device (10) and is configured for conveying the transport containers (2) by means of gravity.

12. Inspection arrangement according to one of claims 1 to 11, wherein
the transport containers (2) are transport bins, and/or
the transport containers (2) each have a contactless identification element, such as an optically detectable code and/or an RFID transponder that can be read by radio.

## Revendications

1. Dispositif d'inspection pour contrôler des objets à inspecter, comme par exemple des bagages à main et d'autres objets transportés par des personnes, comprenant :
un point de dépose (20) sur lequel un conteneur de transport (2) peut être posé et rempli d'un ou plusieurs objets à inspecter ;
un point de retrait (30) où le ou les objets à inspecter peuvent être retirés du conteneur de transport (2),
un dispositif d'inspection (10), par exemple un dispositif d'inspection à rayons X, pour inspecter sans les abîmer le ou les objets à inspecter, le dispositif d'inspection (10) étant entre l'endroit où on pose le conteneur (20) et l'endroit où on enlève le conteneur (30),
un dispositif d'acheminement (12) qui traverse l'installation d'inspection (10) pour faire passer les conteneurs de transport (2) à travers l'installation d'inspection (10) le long d'un trajet (15) qui va du point d'appui (20) jusqu'au point de retrait (30), et
un dispositif de désinfection pour désinfecter automatiquement le conteneur de transport (2) qui est destiné au transport d'un ou de plusieurs objets à inspecter à travers l'installation d'inspection (10) et à leur inspection non destructive dans l'installation d'inspection (10),
le dispositif de désinfection comprenant :
un point d'entrée (40a) où les conteneurs de transport (2) vides arrivent et qui est relié au point de prélèvement (30) du dispositif d'inspection, où les objets à inspecter peuvent être retirés des conteneurs de transport (2),
un point de sortie (40b) relié au point de dépose (20) du dispositif d'inspection, sur lequel les conteneurs de transport (2) peuvent être déposés et remplis avec un ou plusieurs objets à inspecter, et
au moins un dispositif de retour (14) pour transporter les conteneurs de transport (2) vides sur un trajet de retour (16) qui va du point d'entrée (40a) jusqu'au point de sortie (40b), et
au moins un dispositif de désinfection automatique (40) disposé sur le trajet de retour (16) pour désinfecter les conteneurs de transport (2) vides,
le dispositif de désinfection (40) ayant au moins une unité de rayonnement UV (42) pour irradier les conteneurs de transport (2) vides, et
**caractérisé en ce que** le dispositif de désinfection (40) a aussi une unité de commande (56) qui est faite pour contrôler l'activation du dispositif de désinfection (40) en fonction du degré de contamination des surfaces des conteneurs de transport individuels (2).

2. Dispositif d'inspection selon la revendication 1, dans lequel la au moins une unité de rayonnement (41) a une ou plusieurs unités de lumière UV (42) ainsi qu'un ou plusieurs réflecteurs (43) qui sont placés pour irradier les surfaces des conteneurs de transport (2), de préférence toute la surface intérieure et extérieure des conteneurs de transport (2).

3. Dispositif d'inspection selon la revendication 1 ou 2, le dispositif de désinfection (40) ayant au moins une unité de pulvérisation (44) avec au moins une buse de pulvérisation (45) qui est placée pour pulvériser les surfaces des conteneurs de transport (2) avec une solution désinfectante liquide ou sous forme de vapeur, de préférence sur toute la surface intérieure et extérieure des conteneurs de transport (2).

4. Dispositif d'inspection selon la revendication 3, le dispositif de désinfection (40) ayant en plus un système de brosses (46) avec des brosses statiques et/ou rotatives qui sont placées pour agir mécaniquement sur la surface des conteneurs de transport (2) qui a été aspergée de solution désinfectante.

5. Dispositif d'inspection selon la revendication 3 ou 4, le dispositif de désinfection (40) ayant au moins une unité de rayonnement thermique (47) pour irradier et évaporer la solution désinfectante sur la surface pulvérisée ou vaporisée des conteneurs de transport (2).

6. Dispositif d'inspection selon l'une des revendications 3 à 5, le dispositif de désinfection (40) ayant au moins une unité de séchage (48) pour sécher la solution désinfectante restée sur les surfaces des conteneurs de transport (2), l'unité de séchage (48) ayant au moins un appareil de chauffage (48a) et/ou un ventilateur (48b), de préférence un ventilateur à air chaud.

7. Dispositif d'inspection selon l'une des revendications 1 à 6, le dispositif de retour (14) passant à travers le dispositif de désinfection (40).

8. Dispositif d'inspection selon l'une des revendications 1 à 7, le dispositif de désinfection (40) ayant aussi un dispositif de basculement (54) pour changer la position des récipients de transport (2) pendant un processus de désinfection.

9. Dispositif d'inspection selon l'une des revendications 1 à 8, la section de retour (16) s'étendant au moins en partie sous ou à côté du dispositif d'acheminement (12) traversant l'installation d'inspection (10) et comportant en outre un dispositif de levage (50) qui soulève les conteneurs de transport (2) de la section de retour (16) vers une section de transfert (52) pour les conteneurs de transport (2), qui est parallèle au point d'appui (20).

10. Dispositif d'inspection selon la revendication 9, le dispositif de désinfection (40) étant disposé sur la section de retour (16) en dessous ou à côté du dispositif d'amenée (12) et/ou dans le dispositif de levage (50).

11. Dispositif d'inspection selon l'une des revendications 1 à 8, où le tronçon de retour (16) s'étend en tout ou en partie sous forme de plan incliné à côté du moyen d'acheminement (12) menant à l'appareil de contrôle (10) et est conçu pour acheminer les récipients de transport (2) par gravité.

12. Dispositif d'inspection selon l'une des revendications 1 à 11, dans lequel
les récipients de transport (2) sont des bacs de transport, et/ou
les récipients de transport (2) ont chacun un élément d'identification détectable sans contact, comme par exemple un codage détectable optiquement et/ou un transpondeur RFID lisible par radio.
